# EUROPEAN PATENT APPLICATION

(11) **EP 4 599 841 A1**
(43) Date of publication of application: **13.08.2025**
(21) Application number: 24382120.4
(22) Date of filing: 07.02.2024
(51) Int. Cl.: A61K 38/17, A61P 35/00

(54) **PEPTIDES FOR USE IN A METHOD FOR THE TREATMENT OF CANCER**

(71) Applicant: Universidad de Murcia, 30003 Murcia (ES)
(72) Inventor: VALDOR ALONSO, Rut, 30120 Murcia (ES)
(74) Representative: Hoffmann Eitle

(57) **Abstract**

The present invention refers to specific peptides, or pharmaceutical compositions comprising thereof, for use in a method for the treatment of cancer.

## Description

### FIELD OF THE INVENTION

The present invention refers to the medical field. Particularly, the present invention refers to specific peptides, or pharmaceutical compositions comprising thereof, for use in a method for the treatment of cancer.

### STATE OF THE ART

Pericytes, which were first discovered in the 1870s, are described as cells embedded between the basement membrane and endothelial cells of capillaries. They were named pericytes in the 1920s when researchers observed that the endothelial cells of the capillaries were usually surrounded by pericytes, where they could regulate the vasoconstriction response. Further research showed that pericytes, together with endothelial cells, the basement membrane, and vascular smooth muscle cells, constitute the basic structure of human capillaries. Pericytes play an important role in forming the vascular wall, maintaining the vascular barrier, ensuring the stability of blood vessels, and participating in the maintenance of homeostasis in the human body.

The TME refers to the internal and external environment of tumor cells during their growth and metastasis. The composition of the TME is related to different tumor tissue types, and generally includes tumor cells, immune cells, blood vessels, the extracellular matrix, and bioactive molecules. Furthermore, the TME affects the occurrence, development, and metastasis of malignant tumors, and induces immune suppression, immune tolerance, and tumor angiogenesis.

As a component of the TME, pericytes interact with and mutually influence the TME. It is mainly reflected in the following six aspects [1]: Pericytes deviate from endothelial cells and vascular basement membrane in the TME, and the phenotype changes simultaneously. The change of pericytes could damage the local vascular barrier and facilitate the budding proliferation of endothelial cells and tumor angiogenesis. In the later stages of malignancy, these abnormal blood vessels form the hypoxic TME, promoting immune tolerance and affecting the therapeutic efficacy of chemotherapeutic drugs. [2] Before tumor metastasis, pericytes at the secondary sites can detach from vessels, gain phenotypic transformation, and increase the synthesis of extracellular matrix to create a premetastatic niche conducive to the colonization and invasion of hematogenous metastatic cancer cells. [3] Pericytes and cancer cells influence each other through paracrine effects to promote pericyte aggregation and angiogenesis, as well as immune tolerance and drug tolerance of cancer cells. [4] In the TME, the disorder of various signalling pathways makes it difficult for pericytes to recruit and play normal physiological functions, thus making the new vessels show the characteristics of immature and high permeability and promoting the formation of the hypoxic and acidic microenvironment in malignant tumor. [5] There is a bi-directional dialogue between pericytes and tumor-associated macrophages (TAMs). Pericytes can induce the infiltration and polarization of M2 phenotype macrophages, while TAMs can maintain the pericyte-like phenotype of mesenchymal stem cells in the vascular niche and secrete cytokines conducive to angiogenesis. [6] The impaired vessels result in forming the hypoxic TME which enables the production of various EMT-induced signals and stimulates the epithelial-mesenchymal transformation (EMT) of cancer cells. The transformed cancer cells also exhibit the phenotype and function of pericytes, clustering into local blood vessels and causing the occurrence of tumor metastasis.

On the other hand, there is a lack of knowledge of the pathogenesis and mechanisms of progression different types of cancer leading to ineffective therapeutic strategies. For instance, glioblastoma is the most aggressive brain cancer as it is one of the most difficult cancers to treat due to the immune scape of the tumor cells which infiltrate and spread in the brain parenchyma surrounding microblood vessels where pericytes (PCs) are located, supporting tumor growth.

Since there is an unmet medical need of finding reliable strategies for treating cancer, the present invention is focused on solving this problem and an innovative therapeutical strategy, based on the above explained interaction between pericytes and tumor cells, is herein provided.

### DESCRIPTION OF THE INVENTION

The present invention refers to specific peptides, or pharmaceutical compositions comprising thereof, for the treatment of cancer.

Particularly, the therapy strategy provided by the present invention refers to the use of specific peptides (hereinafter "peptides of the invention") or a pharmaceutical composition comprising thereof. The peptides of the invention comprises or consists of SEQ ID NO: l (RIHMVYSKRSGKPRGYAFIEY) or SEQ ID NO: 2 (IHMVYSKRSGKPRGYAFIEY), analogues, or salt forms thereof. The present invention also includes conservative mutations of the peptides of the invention and also peptides having homology/identity of at least 90% with the peptide of the invention.

The preferred candidates may comprise analogues of the peptides of SEQ ID NO: 1 or SEQ ID NO: 2. For instance, they may have a phosphoserine residue in at least one of the serine positions, preferably a phosphoserine residue at position 10 or 7 or both of them:
RIHMVYSKRS(PO₃H₂)GKPRGYAFIEY
RIHMVYS(PO₃H₂)KRSGKPRGYAFIEY
RIHMVYS(PO₃H₂)KRS(PO₃H₂)GKPRGYAFIEY
   or at position 9
IHMVYSKRS(PO₃H₂)GKPRGYAFIEY

On the other hand, the methionine at position 4 or at position 3 may be oxidised, giving rise to the following structures:
RIHM(O)VYSKRSGKPRGYAFIEY
IHM(O)VYSKRSGKPRGYAFIEY

The peptide may comprise both phosphoserine and oxidized methionine residues:
RIHM(O)VYSKRS(PO₃H₂)GKPRGYAFIEY
IHM(O)VYSKRS(PO₃H₂)GKPRGYAFIEY
RIHM(O)VYS(PO₃H₂)KRSGKPRGYAFIEY
IHM(O)VYS(PO₃H₂)KRSGKPRGYAFIEY
RIHM(O)VYS(PO₃H₂)KRS(PO₃H₂)GKPRGYAFIEY
IHM(O)VYS(PO₃H₂)KRS(PO₃H₂)GKPRGYAFIEY

"M(O)" represents oxidized methionine and "S(PO₃H₂)" represents phosphoserine.

Moreover, the peptides of the invention may comprise an acetylation of one or both of the lysine residues at position 8 and/or 12 (SEQ ID NO: 1) or at position 7 and/or 11 (SEQ ID NO: 2).

On the other hand, the peptides have a N-terminus and a C-terminus. The N-terminus (also known as the amino-terminus, NH₂-terminus, N-terminal end or amine-terminus) is the start of a protein or polypeptide, referring to the free amine group (-NH₂) located at the end of a polypeptide. The C-terminus (also known as the carboxyl-terminus, carboxy-terminus, C-terminal tail, carboxy tail, C-terminal end, or COOH-terminus) is the end of an amino acid chain (protein or polypeptide), terminated by a free carboxyl group (-COOH).

Such as it is shown in **Example 2,** a peptide which comprises or consists of SEQ ID NO: l (RIHMVYSKRSGKPRGYAFIEY) with a phosphoserine (pS) (PO₃H₂) residue at position 10 (RIHMVYSKRpSGKPRGYAFIEY) is assayed as proof-of-concept (P140). The assayed peptide reduces glioma cells-PC interactions required for glioma cells proliferation and survival **(Example 2.1)** and the intravenous therapy with the peptide of the invention promotes tumor cell elimination **(Example 2.2).**

So, the goal of the present invention was finding therapeutic strategies able to prevent interactions between glioma cells and PCs (as proof-of-concept) and, therefore, promoting PC anti-tumor functions, as a possible treatment against Cerebral Nervous System tumours like glioma, glioblastoma (GB) or paediatric gliomas.

However, since there are also influences and interactions between PCs and other cancer cells, beyond glioma cells, the therapeutic strategy of the present invention can be extrapolated to other types of cancer caused by said mechanism of action.

Other examples of cancer diseases wherein the interaction of PC and cancer cells plays an important role are, for instance: Pancreatic cancer [Wu, Zhichong et al. "Pericyte stem cells induce Ly6G+ cell accumulation and immunotherapy resistance in pancreatic cancer." EMBO reports vol. 24,4 (2023): e56524. doi:10.15252/embr.202256524], colon and bowel cancer [Mustafa, A et al. "Potential crosstalk between pericytes and cathepsins in the tumour microenvironment. "Biomedicine & pharmacotherapy = Biomedecine & pharmacotherapie vol. 164 (2023): 114932. doi:10.1016/j.biopha.2023.114932], liver cancer [Li, Xiaobo et al. "TCAF2 in Pericytes Promotes Colorectal Cancer Liver Metastasis via Inhibiting Cold-Sensing TRPM8 Channel. "Advanced science (Weinheim, Baden-Wurttemberg, Germany) vol. 10,30 (2023): e2302717. doi:10.1002/advs.202302717], bone cancer [Chang, Le et al. "Pericytes in sarcomas of bone." Medical oncology (Northwood, London, England) vol. 32,7 (2015): 202. doi:10.1007/s12032-015-0651-6], breast cancer and brain tumours [Ippolitov, Danyyl et al. "Brain Microvascular Pericytes More than Bystanders in Breast Cancer Brain Metastasis." Cells vol. 11,8 1263. 8 Apr. 2022, doi:10.3390/cells11081263], mouth cancer [do Valle, Isabella Bittencourt et al. "The participation of tumor residing pericytes in oral squamous cell carcinoma. "Scientific reports vol. 13,1 5460. 4 Apr. 2023, doi:10.1038/s41598-023-32528-1], lung cancer [Bagley, Rebecca G et al. "Pericytes from human non-small cell lung carcinomas: an attractive target for anti-angiogenic therapy." Microvascular research vol. 71,3 (2006): 163-74. doi:10.1016/j.mvr.2006.03.002], melanoma [Jose Humberto Trevino-Villarreal et al., Effect of pericytes or melanoma development.. JCO 30, 83-83(2012).DOI:10.1200/jco.2012.30.30_suppl.83] or renal cancer [Cao, Yun et al. "Pericyte coverage of differentiated vessels inside tumor vasculature is an independent unfavorable prognostic factor for patients with clear cell renal cell carcinoma. "Cancer vol. 119,2 (2013): 313-24. doi:10.1002/cncr.27746].

This means that, the therapeutic strategy of the present invention could be used in the treatment of all types of cancer wherein the interaction of PC and cancer cells plays an important role, as documented above.

So, the first embodiment of the present invention refers to a peptide selected from the group consisting of SEQ ID NO: l or SEQ ID NO: 2, or a peptide having an identity of sequence of at least 95% with SEQ ID NO: l or SEQ ID NO: 2, for use in the treatment of cancer.

The second embodiment of the present invention refers to a pharmaceutical composition comprising a peptide selected from the group consisting of SEQ ID NO: l or SEQ ID NO: 2, or a peptide having an identity of sequence of at least 95% with SEQ ID NO: l or SEQ ID NO: 2, and, optionally, pharmaceutically acceptable excipients or carriers, for use in the treatment of cancer.

In a preferred embodiment, the cancer disease to be treated is promoted by the interaction of pericytes and cancer cells, and the treatment comprises preventing interactions between cancer cells and pericytes and thus, to let pericytes to induce their anti-tumor properties and enhance the anti-tumor immune responses.

In a preferred embodiment, the cancer disease to be treated is Cerebral Nervous System tumours, pancreatic cancer, colon and bowel cancer, liver cancer, bone cancer, breast cancer, brain tumours, mouth cancer, lung cancer, melanoma or renal cancer.

In a preferred embodiment, the cancer disease to be treated is glioma, glioblastoma or paediatric gliomas.

In a preferred embodiment, at least one serine is phosphorylated and/or the methionine is oxidised in the SEQ ID NO: l or SEQ ID NO: 2.

In a preferred embodiment, the serine at position 10 of SEQ ID NO: 1, or the serine at position 9 of SEQ ID NO: 2, is phosphorylated comprising a phosphoserine residue at said position.

In a preferred embodiment, the pharmaceutical composition is administered at a dosage of from about 100 ng to about 5 mg, preferably from about 25 ng a 5 mg.

Alternatively, the present invention refers to a method for treating the above cited types of cancer which comprises administering a therapeutically effective dose or amount of the pharmaceutical composition comprising the peptide/s of the invention.

Any route of administration may be used, for instance systemic or local administration, although intravenous administration is preferred.

For the purpose of the present invention the following terms are defined:
- The expression cancer disease "promoted by the interaction of pericytes and tumor cells" refers to any cancer disease wherein tumor cells interact with pericytes to condition them on their own benefit needed to proliferate and survive.
- The term "comprising" means "including", but not limited to what follows the term "comprising". Thus, the use of the term "comprising" indicates that the elements listed are necessary or mandatory, but that other elements are optional and may or may not be present.
- The term "consisting of" means "including" but is limited to what follows the term "consisting of". Thus, the term "consists of" indicates that the elements listed are mandatory, and that other elements may not be present.
- By "therapeutically effective dose or amount" of a composition comprising the peptide of the invention is intended an amount that, when administered as described herein, brings about a positive therapeutic response in a subject suffering from cancer. The exact amount required will vary from subject to subject, depending on the species, age, and general condition of the subject, the severity of the condition being treated, mode of administration, and the like. An appropriate "effective" amount in any individual case may be determined by one of ordinary skill in the art using routine experimentation, based upon the information provided herein.
- The term "pharmacological composition," "therapeutic composition," "therapeutic formulation" or "pharmaceutically acceptable formulation" can mean, but is in no way limited to, a composition or formulation that allows for the effective distribution of the peptide provided by the invention.
- The term "pharmaceutically acceptable" or "pharmacologically acceptable" can mean, but is in no way limited to, entities and compositions that do not produce an adverse, allergic, or other untoward reaction when administered to an animal, or a human, as appropriate.
- The term "pharmaceutically acceptable carrier" or "pharmacologically acceptable carrier" can mean, but is in no way limited to, any and all solvents, dispersion media, coatings, antibacterial and antifungal agents, isotonic and absorption delaying agents, and the like, compatible with pharmaceutical administration. Suitable carriers are described in the most recent edition of Remington's Pharmaceutical Sciences, a standard reference text in the field, which is incorporated herein by reference. Preferred examples of such carriers or diluents include, but are not limited to, water, saline, finger's solutions, dextrose solution, and 5% human serum albumin. Liposomes and non-aqueous vehicles such as fixed oils may also be used. The use of such media and agents for pharmaceutically active substances is well known in the art. Except insofar as any conventional media or agent is incompatible with the active compound, use thereof in the compositions is contemplated. Supplementary active compounds can also be incorporated into the compositions.
- The term "systemic administration" refers to a route of administration that is, e.g., enteral or parenteral, and results in the systemic distribution of an agent leading to systemic absorption or accumulation of drugs in the blood stream followed by distribution throughout the entire body. Suitable forms, in part, depend upon the use or the route of entry, for example oral, transdermal, or by injection. Such forms should not prevent the composition or formulation from reaching a target cell (i.e., a cell to which the negatively charged polymer is desired to be delivered to). For example, pharmacological compositions injected into the blood stream should be soluble. Other factors are known in the art and include considerations such as toxicity and forms which prevent the composition or formulation from exerting its effect. Administration routes which lead to systemic absorption include, without limitations: intravenous, subcutaneous, intraperitoneal, inhalation, oral, intrapulmonary and intramuscular. The rate of entry of a drug into the circulation has been shown to be a function of molecular weight or size. The use of a liposome or other drug carrier comprising the compounds of the instant invention can potentially localize the drug, for example, in certain tissue types, such as the tissues of the reticular endothelial system (RES). A liposome formulation which can facilitate the association of drug with the surface of cells, such as, lymphocytes and macrophages is also useful.
- The term "local administration" refers to a route of administration in which the agent is delivered to a site that is apposite or proximal, e.g., within about 10 cm, to the site of the lesion or disease.
- The term "conservative mutations" refers to the substitution, deletion or addition of nucleic acids that alter, add or delete a single amino acid or a small number of amino acids in a coding sequence where the nucleic acid alterations result in the substitution of a chemically similar amino acid. Amino acids that may serve as conservative substitutions for each other include the following: Basic: Arginine (R), Lysine (K), Histidine (H); Acidic: Aspartic acid (D), Glutamic acid (E), Asparagine (N), Glutamine (Q); hydrophilic: Glycine (G), Alanine (A), Valine (V), Leucine (L), Isoleucine (I); Hydrophobic: Phenylalanine (F), Tyrosine (Y), Tryptophan (W); Sulfur-containing: Methionine (M), Cysteine (C). In addition, sequences that differ by conservative variations are generally homologous.
- By "homology" is meant the nucleotide sequence of two or more nucleic acid molecules or two or more nucleic acid or amino acid sequences is partially or completely identical.

In certain embodiments the homologous nucleic acid or amino acid sequence has 30%, 40%, 50%, 60%, 70%, 80%, 90%, or 95% sequence similarity or identity to a nucleic acid encoding the amino acid sequence of SEQ ID NO: 1 or SEQ ID NO:2, respectively.

"Homologs" can be naturally occurring or created by artificial synthesis of one or more nucleic acids having related sequences, or by modification of one or more nucleic acid to produce related nucleic acids. Nucleic acids are homologous when they are derived, naturally or artificially, from a common ancestor sequence (e.g., orthologs or paralogs).

If the homology between two nucleic acids is not expressly described, homology can be inferred by a nucleic acid comparison between two or more sequences. If the sequences demonstrate some degree of sequence similarity, for example, greater than about 30% at the primary amino acid structure level, it is concluded that they share a common ancestor. For purposes of the present invention, genes are homologous if the nucleic acid sequences are sufficiently similar to allow recombination and/or hybridization under low stringency conditions. In addition, polypeptides are regarded as homologous if their nucleic acid sequences are sufficiently similar to allow recombination or hybridization under low stringency conditions, and optionally they demonstrate membrane repair activity, and optionally they can be recognized by (i.e., cross-react with) an antibody specific for an epitope contained within the amino acid sequence of at least one of SEQ ID NOs: 1 or 2.
- The term "analogues" can mean, but is in no way limited to, chemical compositions, for example, nucleic acids, nucleotides, polypeptides or amino acids that have a structure similar to, but not identical to, the native compound.

### Description of the figures

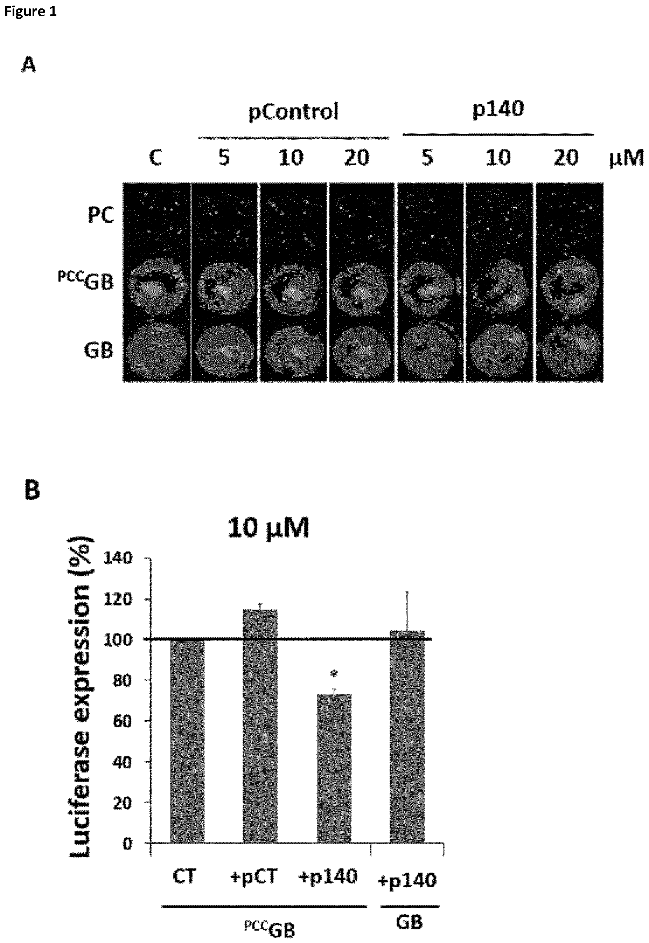

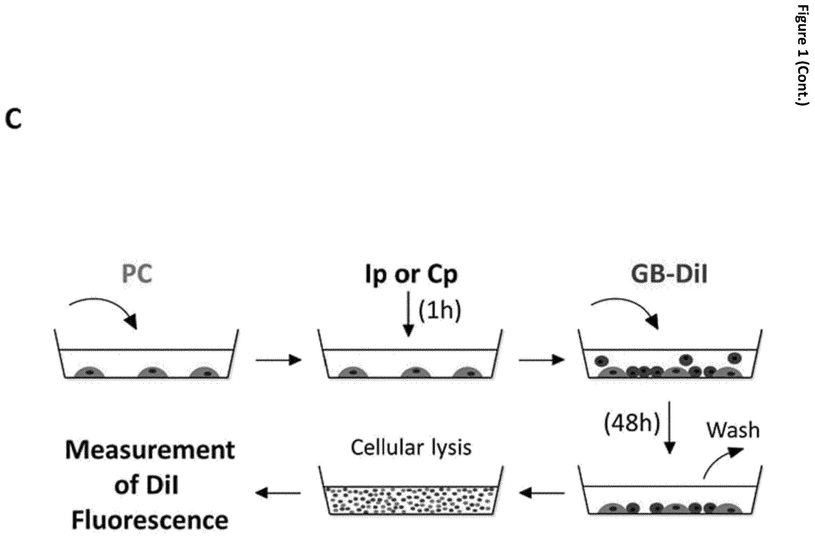

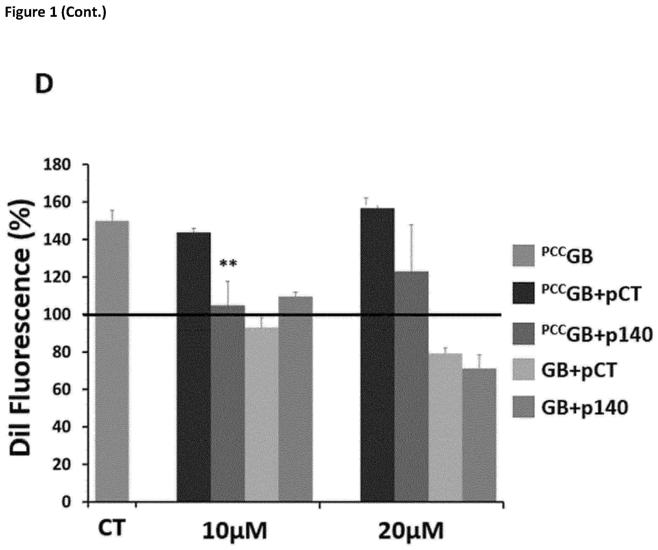

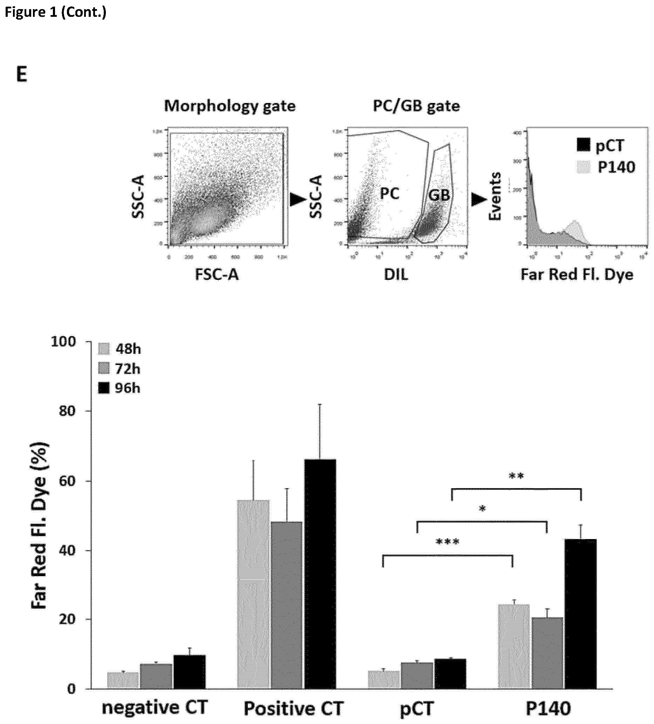

**Figure 1****. The CMA inhibitor peptide reduces GB-PC interaction required for GB proliferation and survival. (A-E)** Proliferation, adhesion and viability assay: GB cells alone were treated with CMA inhibitor (P140) or control peptide (scramble, from peptide 140, i.e., a peptide with the same sequence but with different positions of the amino acid residues) (pCT) or cocultured (1:1) with peptide-pretreated PCs (^{PCC}GB) with several concentrations for 48 hours. **(A)** Representative figure of luminescence related to proliferation of GB-U87luciferase cell line. **(B)** Quantification of luminescence for 10 mM of P140. Means ± standard error of at least, 3 experiments are shown, * p<0,05 expressed as fold over ^{PCC}GB control. **(C)** Scheme of cell adhesion assay. **(D)** RFP fluorescence in the wells (belonging to the GB that adhered to PC) was measured at 488 nm. Graph represents percentage of adhesion relative to fluorescent levels of control GB cells alone of at least 5 experiments with several GB cell lines ** p<0,01; **(E)** Detection of GB cell death by LIVE/DEAD far red-fluorescent reactive dye (far red-fluorescent dye), following coculture for different time points with pretreated or not PCs (CT) with pCT or P140 and compared to cell death in GB cells cocultured with NAc-treated PC as positive control. Gating strategy to identify DiD+ GB-labeled cell population is shown (Left). Representative flow cytometry histogram and bar graph showing quantification of dead cell percentages from at least 4 independent experiments with different GB cell lines, *P < 0.05, **P < 0.01 or ***P < 0.001.

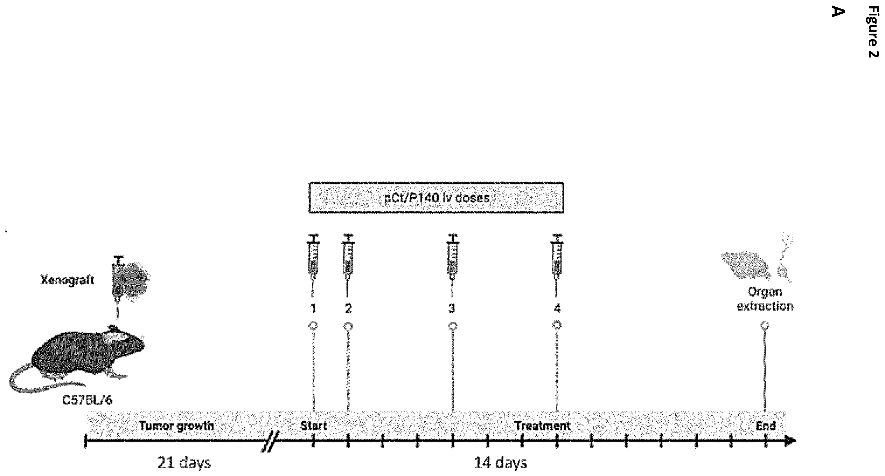

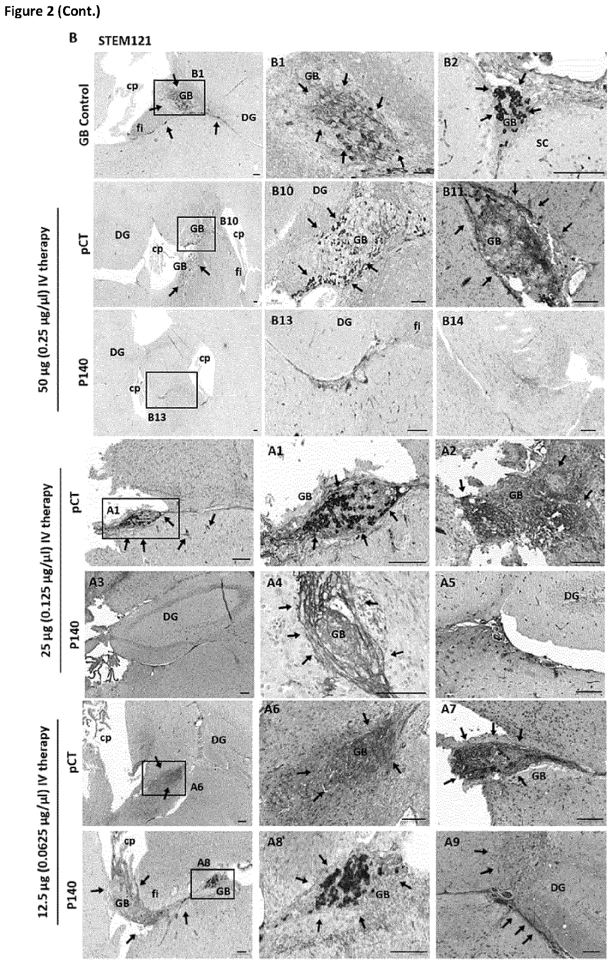

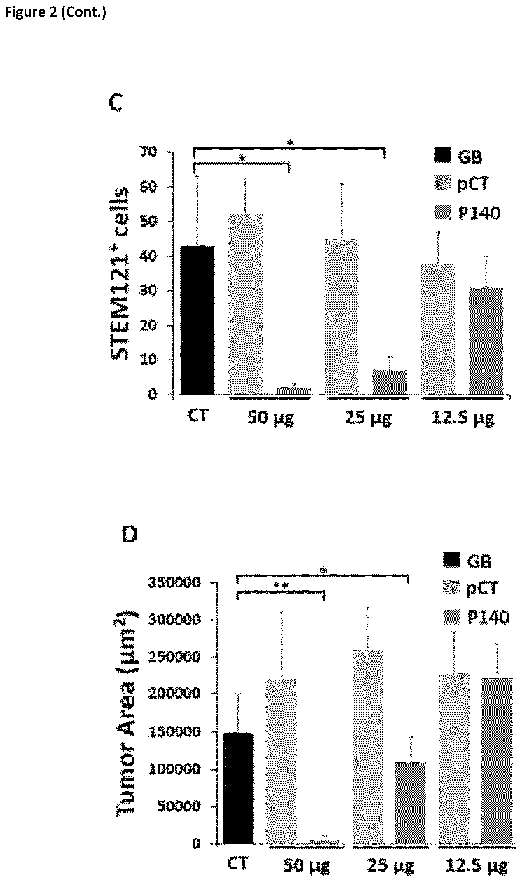

**Figure 2****. Intravenous therapy with the peptide 140 promotes tumor cell elimination. (A)**Timeline of xenografts and treatment. Three weeks after xenografts, mice were treated intravenously with four doses of control peptide (pCT) or peptide 140 (P140). The first two doses were applied on consecutive days and the last two were administered every three days. One week after the last dose, mice were euthanized, and organs were extracted. **(B)** Xenografts of GB tumor cells from mice intravenously treated with different concentrations of control peptide (pCT) and peptide 140 (P140). STEM121 was used to stain GB cells. Control GB brains without treatment show tumor cell mass and tumor cell infiltration (arrows in B1,2). The dose-response of 3 peptide concentrations (0.25, 0.125 and 0.0625 ug/ul) was analyzed. Control peptide therapy did not affect tumor cell infiltration at any concentration (B3, 4, 7, 8, 12, 13), showing tumor proliferation around dentate gyrus (DG), choroid plexus (cp) and fimbria (fi), as in controls. In P140 therapy grafted brains, GB cells were not detected at 0.25 ug/ul dose (B5, B6) while tumor masses started to be detected as the peptide concentration was reduced (B10, 14, 15). Scale bars: 100 µm. All results are shown using the U87 GB line, 4 weeks after therapy, and are representative of at least three independent experiments using both U87 or U251 GB cell lines, independently. **(C)** Relative quantification of STEM 121 positive cells related to tumor cells per brain. **(D)** Morphometric measurement of tumor size related to the surface area of the tumor cell mass. All results are mean+SD from at least three independent experiments using both U251 and U87 GB lines, independently; *p < 0.05, **p < 0.01.

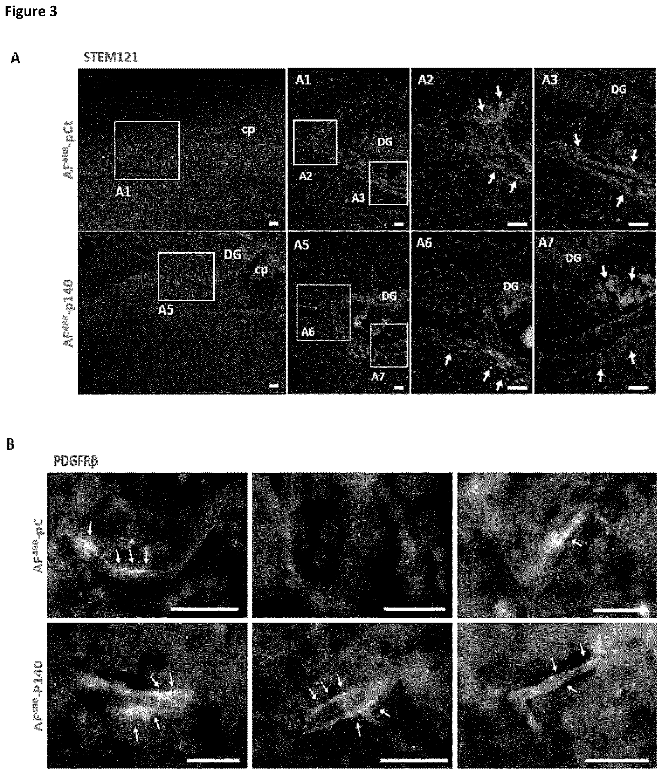

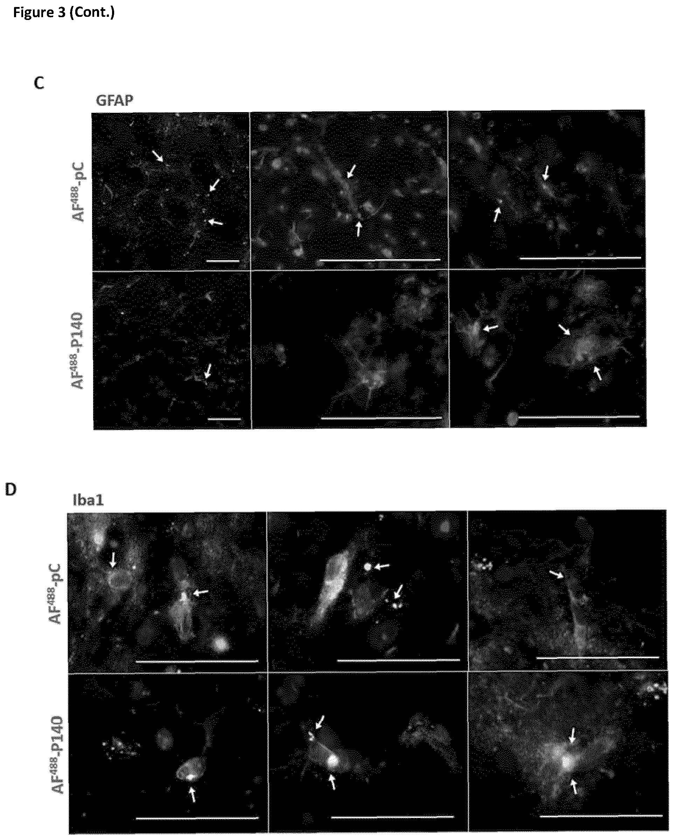

**Figure 3****. The peptide 140 reaches tumor niche and accumulates in peritumoral areas as the control peptide.** Control peptide (pCT) or peptide 140 (P140) labelled with AlexaFluor-488 (AF488) was injected intravenously to 6 GB-xenografted mice to control their localization in the brain after 24h (Dentate gyrus (DG); Choroid plexus (cp)). **(A)** Peptide accumulation (in green) was analyzed in the tumor niche using STEM121 to stain GB cells (in red). **(B)** Colocalization of the peptide with PCs was identified staining the pericyte marker PDGFRβ (in red). **(C)** Colocalization of the peptide with astroglia cells was identified using the astrocytes marker GFAP. **(D)** Colocalization with microglia cells was detected by Iba1 expression. Scale bars: 100 µm.

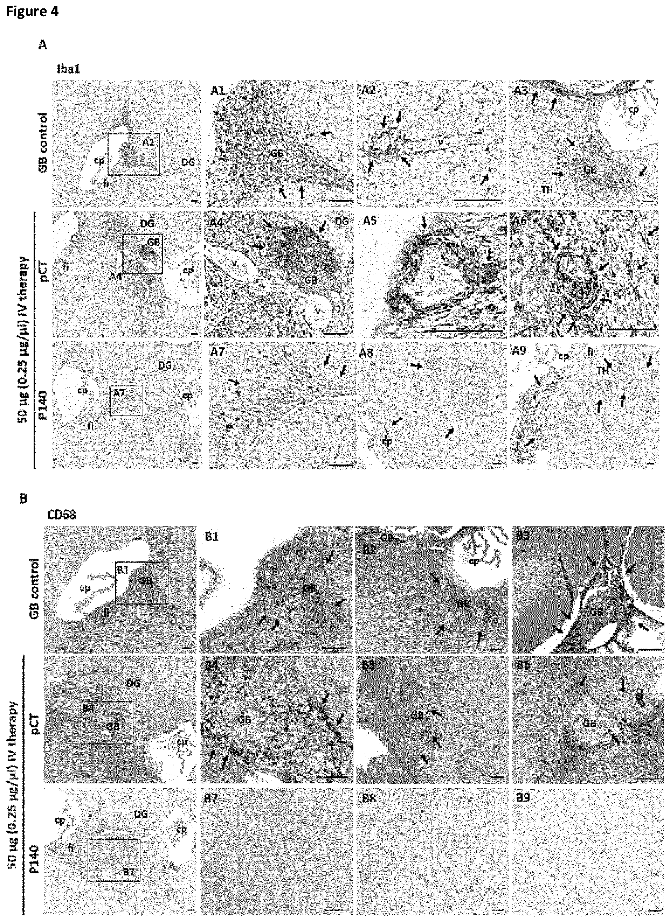

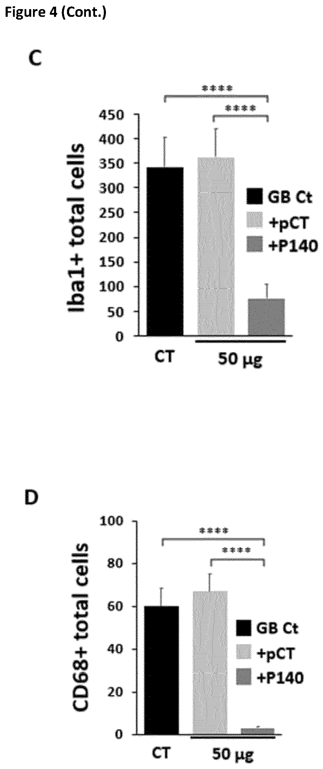

### Figure 4. Phagocytic populations associated to the tumor are reduced in GB mice treated with peptide 140.

Iba-1 immunopositive cells are abundant in tumor areas **(A1)** and host peritumoral brain parenchyma **(A1, 2, 3).** pCT therapy shows infiltration of activated microglia associated to the tumor cell areas **(A4-6).** The P140 therapy shows accumulation of microgliosis (arrows in **A7-9)** around grafted areas and close to previous tumorigenesis that was eliminated around the thalamus (TH). **(B)** CD68 immunostaining exhibited positive results only in GB control tumor graft areas **(B1-B3).** Similar staining is shown in pCT therapy **(B4-B6)** whereas CD68⁺ cells were hardly detected with P140 therapy **(B7-B9).** Blood vessel (v); Dentate gyrus (DG); fimbria (fi); Choroid plexus (cp). Scale bars: 100 µm. **(C)** Relative quantification of Iba-1 immune-positive particles (pixels) in total brain. **(D)** Quantification of CD68 positive cells in total brain. All results are mean+SD from at least, three independent experiments using both U251 and U87 GB lines, independently; ***p < 0.001.

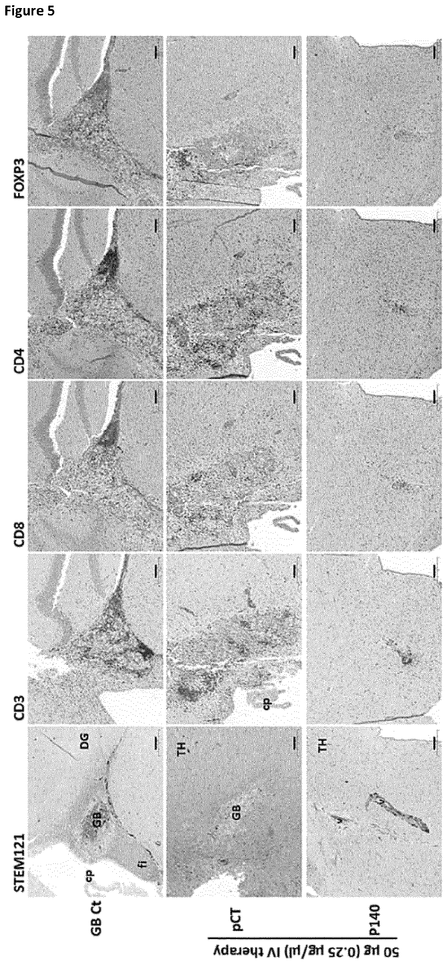

### Figure 5. Brain infiltration of T-cell populations associated to the tumor are reduced in GB mice treated with peptide 140.

Characterization of infiltrating T cells marked with CD3, CD8, CD4 and FOXP3 in xenografts. GB cells were stained with STEM121. Images show infiltration of CD3+ cells in control and pCT treated mice, while P140 treated mice just show remaining T cells after tumor elimination. CD8⁺ and CD4⁺ tumor-associated T cells, including those expressing FOXP3⁺, were found in intratumoral areas, showing no differences between GB control and pCT treated mice. P140 treated mice showed that the major remaining T cells were CD8⁺ with hardly detection of FOXP3 expression. Scale bars: 100 µm.

### Detailed description of the invention

The present invention is illustrated by means of the Examples set below without the intention of limiting its scope of protection.

### Example 1. MATERIAL AND METHODS

### Example 1.1. Isolation and coculture

[1:1] of murine brain pericytes from C57BL/6 or C57BL/6-Tg (ACTB-EGFP)1Osb/J (Charles River Laboratory) mice and human GB cell lines (U87, U251 and U373) for different time points kinetic was done following the methods already described [Valdor et al., 2017, Oncotarget] .

### Example 1.1. Proliferation Assays

Luciferase activity was measured in GB-U87 cell co-cultured during 48 hours with pretreated PC-GFP cells with or without (C) control peptide (pC) or inhibitor peptide (pI) at several concentrations and 1 hour previously to add Glioblastoma cells. The effect of the peptides in GB-U87 cells alone was compared.

### Example 1.2. Adhesion assays

GB-U251, -U273 labeled with DiI (cell tracker, Thermophiser), or GB-U87 cells expressing RedFluorescenceProtein, were cocultured on a monolayer culture of PCs for 48 h. Cells were lysed after washing away non adhered cells with media, and remaining RFP fluorescence in the wells (belonging to the GB that adhered to PC) was measured at 488 nm (588-nm. emission).

### Example 1.3. Survival assays

GB cells were analyzed using DiI labeling solution for tracking and separation of cells. GB cell death was determined using LIVE/DEAD Fixable far red Dead Cell Stain Kit, (ThermoFisher). Stained cells were analyzed by flow cytometry using a FACSCanto flow cytometer (BD Bioscience) and data were analyzed with Kaluza analysis software (Beckman Coulter).

### Example 1.4. Mice

Male and female mice of eight to 12 weeks old WT C57BL/6 (Charles River Laboratories) were maintained in pathogen-free conditions in the animal facilities of the Biomedical Research Institute of Murcia Virgen de la Arrixaca. All animal procedures were approved and performed according to the guidelines set by the Institutional Animal Care and Use Committee.

### Example 1.5. Xenografts of human GB cells and therapy

Cell pellets (5 × 10⁶ cells) from human GB cells (U87 and U251 GB lines) were grafted into C57BL/6 wild type mice brains. Xenografts were performed as previously described in an immunocompetent mouse model [Molina et al., Front.cell.Dev., 2022]. Cell pellets, prepared as hanging drops, were grafted into mice. Xenografts (1 pellet/mouse) were introduced into the right hemisphere through a small craniotomy (2-3 mm from the midline, approximately 1 mm behind the bregma) at 2.5 mm depth, using a stereotactic apparatus and a Pasteur pipette hand-pulled to an internal diameter of 0.38 mm. This produced grafts that integrated into the occipital cortex or the hippocampus. Three weeks post-grafting, after tumor cell mass was established, mice were treated intravenously through tail injection with different doses of the peptide (4 doses of 50 mg each per mouse: 10 mg/kg, 4 doses of 25 mg per mouse: 5 mg/kg or 4 doses of 12.5 mg per mouse: 2.5 mg/kg) to analyze a dose response comparing to those none treated or treated with a control peptide. Four weeks after therapies, mice were sacrificed, and brains were fixed in 4% buffered formalin (Panreac Quimica). All procedures were repeated four times and analyzed in each experimental line (A total of 20 mice for any experimental line).

### Example 1.6. Immunohistochemistry

Brains were paraffin embedded and processed by the Pathology facility (IMIB Virgen de la Arrixaca) as described previously [Valdor et al., 2019, PNAS]. Three-micrometer thick serial sections were obtained from paraffin embedded samples using an automatic rotary microtome (Thermo Scientific). For colorimetric immunolabeling, sections were incubated overnight at 4°C with mouse anti-human STEM121 (Cellartis), goat anti-Iba-1 (Abcam), rat anti-CD68 (Abdserotec), rabbit anti-CD3 (Dako-Agilent), rabbit anti-CD4 (Abcam), rabbit anti-CD8 (Abcam), rat anti-FOXP3 (EBioscience), primary antibodies. Sections were finally incubated with the corresponding 3-3'Diaminobencidine (DAB) secondary antibodies (Vector Labs) and hematoxylin counterstained. Positive immunoreaction was identified as a dark-brown precipitated. An automatic digital slide scanner (Pannoramic MIDI II-3DHistech) and Quantitative Pathology & Bioimage Analysis Qupath-0.2.3 software were used for analysis of histological sections, and acquisition of images. Morphometric measurements and quantification of cells were also performed using Imaged (NIH, United States) software.

All determination were performed in brains of at least n = 5 mice per experimental group and four separate experiments.

### Example 1.7. For Peptide localization and immunofluorescence

Peptide140 or control peptide labeled with Alexa fluor 488 kindly provided by Professor S. Muller [Macri et al., 2015, Autophagy; Monneaux et al. Eur J Immunol 2003; Page et al., PLoS ONE 2009] were injected intravenously (4 doses of 50 mg per mouse) and analyzed by immunofluorescence after 24 fours. For brain fixation, brain tissue immunofluorescence and microscopy, we followed the methods already described [Valdor et al., 2019, PNAS]. Tumor cells were detected with mouse anti-human STEM121 (Cellartis). PCs in blood microvessels of peritumoral areas were identified using a goat anti-mouse antibody (Neuromics) against the pericyte marker PDGFR-b. For astrocytes, a rabbit anti-GFAP antibody (Abcam). For microglia, a goat anti-Iba-1 (Abcam). Anti-goat or rabbit or mouse Cyanine5 (Invitrogen) antibodies were used as secondary antibodies.

### Example 1.8. Statistical Analysis

Differences between groups were analyzed by one-way ANOVA followed by Tukey-Kramer posttest. Comparisons between data pairs were analyzed using a t test. Statistical significance was defined as *p* < 0.05.

### Example 2. RESULTS

### Example 2.1. The peptide 140 reduces GB-PC interactions required for GB proliferation and survival

PCs promote GB survival and proliferation through physical interaction enhancing the immunosuppressive function of PCs that prevent anti-tumor immune responses. We analyzed the GB cell proliferation and survival and the PCs-tumor cell interactions in presence or absence of two different concentrations of the peptide and compared to a control peptide and GB cells alone. Luciferase activity related to tumor cell proliferation and survival was measured in GB-U87 cell co-cultured with pretreated PC with or without (C) control peptide (pC) or peptide140 (pI) at several concentrations and 1 hour previously to add Glioblastoma cells **(****Figure 1A****).** We also compared the effect of the peptides in GB-U87 cells both in the presence and in the absence of PC cells. We found that the effect of the inhibitor peptide significantly reduced the luciferase expression related to GB cell levels in the cocultures PCCGB, and the effect was dependent on the peptide concentration **(****Figure 1A****),** finding as the optimal concentration, 10 µM **(****Figure 1A**, **B**).

To analyze the stable interactions between GB and PC, several GB cell lines stained with a fluorescent cell-tracker (dil) were added on a monolayer of pretreated PC with control or inhibitor peptide and co-cultured for 48 hours. The percentage of adhesion was analyzed related to levels of fluorescence that were normalized to control adhesion of control GB cells **(****Figure 1C****).** Our results corroborated the findings showing reduced GB proliferation in an optimal concentration of 10 µM compared to higher concentration of the peptide and controls. Our results revealed that the inhibitor peptide (pI) significantly reduced the stable cell-to-cell interactions between GB and PC **(****Figure 1D****),** which promote tumor cell proliferation and survival [Valdor et al., 2019, PNAS].

In line with our previous results, we found that the GB cell survival was affected in presence of pretreated PCs with P140 and compared to those nontreated control (negative CT) or treated with the control peptide (pCT) **(****Figure 1 E)****.**

### Example 2.2. Intravenous therapy with peptide 140 promotes tumor cell elimination

To determine if the P140 was also affecting the proliferation and survival of GB cells *in vivo*, GB mice [Molina et al, 2022, Front. Cell. Dev. Biol], were treated intravenously with a P140 or pCT therapy and using different P140 doses to determine a dose-response **(****Figure 2A****).** After, 14 days of treatment, cells of a previously engrafted tumor (GB control) were hardly detected outside of the brain parenchyma in GB mice treated with the highest dose of P140, **(****Figure 2B****;** 50 µg) and compared to those treated with pCT and GB control mice, which showed brain infiltration of tumor cells in choroid plexus and subpial vessels, and tumor masses in brain cortex around perivascular areas. The effect of P140 on the tumor cells resulted dose dependent, as mice treated with lower dose concentration showed an evident reduced tumor mass and remaining tumor cells close to blood vessels whereas, no significant differences were founded with the lowest dose of the peptide **(****Figures 2** **B, C** and **D).**

As we wanted to demonstrate that intravenously injected P140 reached tumor niche, going through the blood brain barrier (BBB), GB-grafted mice were intravenously injected with P140 labeled with Alexa 488 (P140^{AF488}) and compared to those injected with control peptide (pCT^{AF488}) **(****Figure 3****),** using the most efficient dose of P140 (50 µg per mouse). We found both peptides reached tumor areas of the brain cortex, being mainly accumulated in peritumoral areas **(****Figure 3 A)** after 24 hours of injection. Peptides colocalized mainly in perivascular areas with pericytes **(****Figure 3 B)** and in the rest of the brain parenchyma, hardly with astrocytes **(****Figure 3 C)** and mainly with microglia cells **(****Figure 3 D)****.** Importantly, we found that mice treated with P140 did not show phagocytic immune populations associated to the tumor, after the treatment **(****Figure 4****).** The expression of the microglia marker Iba-1 **(****Figure 4 A)** and the macrophages activation marker CD68⁺ **(****Figure 4B****)** were significantly reduced compared to mice untreated or treated with the control peptide **(****Figure 4****).** Moreover, infiltration of different T cells populations associated to the tumor progression were reduced, hardly detecting remaining CD4+ cells without any evidence of infiltration of immunosuppressive cells presenting FOXP3 **(****Figure 5****)** and thus, showing only the presence of effector CD8⁺ T cells against tumor cells.

## Claims

1. Peptide selected from the group consisting of SEQ ID NO: l or SEQ ID NO: 2, or a peptide having an identity of sequence of at least 95% with SEQ ID NO: l or SEQ ID NO: 2, for use in the treatment of cancer.

2. Pharmaceutical composition comprising a peptide selected from the group consisting of SEQ ID NO: l or SEQ ID NO: 2, or a peptide having an identity of sequence of at least 95% with SEQ ID NO: l or SEQ ID NO: 2, and, optionally, pharmaceutically acceptable excipients or carriers, for use in the treatment of cancer.

3. Peptide or pharmaceutical composition for use, according to claims 1 or 2, in the treatment of cancer disease promoted by the interaction of pericytes and cancer cells, wherein the method comprises administering the peptide or pharmaceutical composition of claims 1 or 2 to prevent interactions between cancer cells and pericytes.

4. Peptide or pharmaceutical composition for use, according to any of the previous claims, in the treatment of: Cerebral Nervous System tumours, pancreatic cancer, colon and bowel cancer, liver cancer, bone cancer, breast cancer, brain tumours, mouth cancer, lung cancer, melanoma or renal cancer.

5. Peptide or pharmaceutical composition for use, according to any of the previous claims, in the treatment of: Glioma, glioblastoma or paediatric gliomas.

6. Peptide or pharmaceutical composition for use, according to any of the previous claims, wherein at least one serine is phosphorylated and/or wherein the methionine is oxidised in the SEQ ID NO: l or SEQ ID NO: 2.

7. Peptide or pharmaceutical for use, according to any of the previous claims, wherein the serine at position 10 of SEQ ID NO: 1, or the serine at position 9 of SEQ ID NO: 2, is phosphorylated comprising a phosphoserine residue at said position.

8. Pharmaceutical composition, for use, according to any of the previous claims, wherein the pharmaceutical composition is administered at a dosage of from about 100 ng to about 5 mg, preferably from about 25 ng a 5 mg.
